# EUROPÄISCHE PATENTSCHRIFT

(11) **EP 1 585 438 B1**
(45) Veröffentlichungstag und Bekanntmachung des Hinweises auf die Patenterteilung: **18.10.2006**
(21) Anmeldenummer: 04703395.6
(22) Anmeldetag: 20.01.2004
(51) Int. Cl.: A61B 3/032

(54) **SEHTESTGERÄT ZUR PRÜFUNG DES SEHVERMÖGENS DER AUGEN EINES PROBANDEN**
VISION TESTING DEVICE FOR TESTING THE VISION OF THE EYES OF A TEST PERSON
APPAREIL DE TEST DE VISION SERVANT A VERIFIER L'APTITUDE VISUELLE D'UN SUJET VOLONTAIRE

(30) Priorität: 21.01.2003 DE 10302125; 11.03.2003 DE 10310589
(43) Veröffentlichungstag der Anmeldung: 19.10.2005
(73) Patentinhaber: Vistec AG, 82140 Olching (DE)
(72) Erfinder: REIS, Werner, 80992 München (DE)
(74) Vertreter: Rösler, Uwe
(86) Internationale Anmeldenummer: PCT/EP2004/000428
(87) Internationale Veröffentlichungsnummer: WO 2004/064625

(56) Entgegenhaltungen:
- WO-A-01/49166
- DE-A- 19 540 802
- DE-A- 19 851 715
- DE-B- 1 248 330
- US-A- 2 798 408
- US-A- 5 319 398
- US-A- 5 617 157
- US-A- 5 793 469
- US-A- 6 045 227
- US-A1- 2002 109 819

## Beschreibung

### Technisches Gebiet

Die Erfindung bezieht sich auf ein Sehtestgerät zur Prüfung des Sehvermögens der Augen eines Probanden mit einer optischen Abbildungseinheit zur virtuellen Abbildung eines innerhalb der Brennweite der Abbildungseinheit befindlichen und aus unterschiedlichen Entfernungen abbildbaren Testobjekts in die in der Fokalebene der Abbildungseinheit befindlichen Augen des Probanden, und mit einem im Strahlengang zwischen den Augen des Probanden und der Abbildungseinheit ersten sowie einem zwischen der Abbildungseinheit und dem Testobjekt angeordneten zweiten Element.

### Stand der Technik

Sehtestgeräte der vorstehend genannten Gattung verfügen in aller Regel über einen kompakten und verhältnismäßig leichten Aufbau, so dass sie in Form portabler Untersuchungseinheiten vor Ort, beispielsweise zur Sehtestkontrolle bei Schülern und Studenten in den jeweiligen Ausbildungsstätten oder bei Arbeitnehmern in den jeweiligen Betriebsräumen, eingesetzt werden können. Derartige Sehtestgeräte weisen in aller Regel ein Gehäuse mit Einsichtschlitz auf, in dem der jeweilige Proband zur Augenuntersuchung blickt. Durch Anlegen eines Kopfbereiches, zumeist die Stirn, an einer am Gehäuse vorgesehenen Anlagefläche wird das zu untersuchende Augenpaar in eine definierte Relativlage zum Sehtestgerät gebracht, um letztlich reversible und zuverlässige Messdaten über den jeweiligen Sehzustand der Augen zu erhalten. Hierzu wird ein im Inneren des Sehtestgerätes erzeugbares Testobjekt über eine Abbildungseinrichtung auf die sich in der Referenzebene befindlichen Augen des Probanden fokussiert. Durch Variation des optischen Weges zwischen dem Testobjekt und der Abbildungseinheit kann das Sehvermögen des Probanden in unterschiedliche Sehdistanzen untersucht werden, wobei das natürliche Akommodations- und Konvergenzeinstellverhalten der Augen berücksichtigt werden. Durch die Sehweitenvariation ist es möglich, das Kurz- und Weitsichtigkeitsverhalten der Augen eines Probanden zu überprüfen.

Aus der DE 195 01 415 C2 ist ein derartiges Sehtestgerät zu entnehmen, in dessen Gehäuseinneren zur Erzeugung eines auf die Augen eines Probanden abbildbares Testobjektes eine Durchlichttestscheibe vorgesehen ist, die von einer Lichtquelle vergleichbar einer Dia-Projektoranordnung durchleuchtet wird. Das im Wege der Durchlichtprojektion erzeugte Bild des Testobjektes wird über eine optische Abbildungseinrichtung, die relativ zu der Durchlichtprojektoranordnung stationär angeordnet ist, über einen im Strahlengang der Abbildungseinrichtung nachgeordneten Teilerspiegel auf die Fokalebene, in der sich die Augen des Probanden befinden, abgebildet. Zur Sichtweitenvariation ist zwischen der Durchlichtprojektoranordnung und der optischen Abbildungseinrichtung eine den Strahlengang um 180° umlenkende optische Umlenkeinrichtung vorgesehen, die längs der jeweils um 180° gefalteten Strahlengänge linear beweglich angeordnet ist.

Der von der Durchlichtprojektoranordnung ausgehende Strahlengang zur Erzeugung des Bildes eines Testobjektes ist in dem vorstehend erwähnten Sehtestgerät vertikal die Sehtestscheibe nach unten passierend gerichtet und wird von der Umlenkeinrichtung um 180° senkrecht nach oben umgelenkt, trifft über die Abbildungseinrichtung auf den im Strahlengang befindlichen Teilerspiegel, der den Strahlengang letztlich, je nach Kippstellung, schräg nach oben oder in die horizontale Blickrichtung des Probanden umlenkt.

Demgegenüber sieht eine dem vorstehend beschriebenen Sehtestgerät vorausgegangene Entwicklungsstufe, die in der EP 0 492 044 B1 beschrieben ist, als Abbildungseinrichtung einen mit horizontal orientierter optischer Achse aufweisenden Konkavspiegel vor, innerhalb dessen Brennweite ein Testobjekt verschiebbar angeordnet ist. Darüber hinaus ist im Strahlengang des Konkavspiegels ein teildurchlässiger Spiegel vorgesehen, der das Abbild des Testobjektes um 90° vertikal nach oben auf einen zweiten teildurchlässigen Spiegel ablenkt, der schließlich das Testobjekt scharf in Blickrichtung des Auges eines Probanden, vorzugsweise mit waagrechter Blickrichtung, umlenkt. Auch im Falle des in der EP 0 492 044 B1 beschriebenen Sehtestgerätes wird das Testobjekt im Wege einer Durchlichtprojektion durch eine Testscheibe, auf der eine Vielzahl von Testobjekten, sogenannte Optotypen, aufgebracht sind, erzeugt. Eine Sehweitenvariation wird in diesem Fall durch eine körperliche Verschiebung des Testobjektes, d.h. des gesamten Durchlichtprojektors nebst Testscheibe, realisiert.

### Darstellung der Erfindung

Ausgehend von dem vorstehend genannten Stand der Technik gilt es, ein Sehtestgerät zur Prüfung des Sehvermögens der Augen eines Probanden mit einer optischen Abbildungseinheit zur virtuellen Abbildung eines innerhalb der Brennweite der Abbildungseinheit befindlichen und aus unterschiedlichen Entfernungen abbildbaren Testobjektes in die in der Fokalebene der Abbildungseinheit befindlichen Augen des Probanden und mit einem im Strahlengang zwischen den Augen des Probanden und der Abbildungseinheit ersten sowie einem zwischen der Abbildungseinheit und dem Testobjekt angeordneten zweiten Element, wie es beispielsweise aus der vorstehend zitierten DE 195 01 415 C2 hervorgeht, derart weiterzubilden, dass die für die Überprüfung des Sehvermögens eines Probanden erforderlichen optischen und elektronischen Komponenten auf ein Minimum reduziert und zugleich die Bedien- und Wartungsfreundlichkeit des Sehtestgerätes verbessert werden sollen. Insbesondere gilt es, den Komfort für den zu untersuchenden Probanden während der Überprüfung des Sehvermögens zu verbessern. Auch gilt es vornehmlich bei zumindest gleich bleibender optischer Darstellungsqualität der jeweiligen Testobjekte die mit dem Sehtestgerät verbundenen Kosten erheblich zu reduzieren. Zugleich soll das Sehtestgerät optional modular erweiterbar sein, um über die bloße Sehschärfebestimmung der Augen eines Probanden auch die Farbsichtigkeit oder die perimetrische Sehtüchtigkeit überprüfen zu können.

Die Lösung der der Erfindung zugrunde liegenden Aufgabe ist im Anspruch 1 angegeben. Den Erfindungsgedanken vorteilhaft weiterbildende Merkmale sind Gegenstand der Unteransprüche sowie der Beschreibung, insbesondere unter Bezugnahme auf die Ausführungsbeispiele zu entnehmen.

Das erfindungsgemäße Sehtestgerät gemäß dem Oberbegriff des Anspruches 1 zeichnet sich dadurch aus, dass die den Strahlengang umlenkenden Elemente über nahezu keine Lichtverluste verfügen, zumal sie in bevorzugten Ausführungsformen entweder als Vollspiegel oder als total reflektierende optische Elemente, beispielsweise Prismenflächen, die unter dem Brewster-Winkel relativ zum Strahlengang angeordnet sind, ausgebildet sind. Durch das erste und zweite voll reflektierende optische Element können die vom Testobjekt ausgehenden Lichtstrahlen nahezu verlustfrei in die Fokalebene der Abbildungseinheit, in der sich die zu untersuchenden Augen des Probanden befinden, abgebildet werden. Hierdurch eröffnet sich erstmals die Möglichkeit, in Abkehr vom Stand der Technik, das Bild eines Testobjektes mit Hilfe einer elektronisch ansteuerbaren Monitoreinheit zu generieren und nahezu ohne jegliche Lichtverluste auf die zu untersuchenden Augen des Probanden abzubilden. Die elektrisch ansteuerbare Monitoreinheit, die als klein bauende Komponenten kommerziell erhältlich ist, ist zur Sehweitenvariation längs des durch das zweite Element umgelenkten Strahlengangs beweglich angeordnet.

Mit Hilfe des Einsatzes einer elektronisch ansteuerbaren Monitoreinheit, die beispielsweise in Form eines klein bauenden LCD-Displays mit einer typischen Display-Größe von etwa 10 x 10 mm und einer Pixeldichte von wenigstens 600 x 800 ausgebildet ist, ist es nicht nur möglich, nahezu beliebig ausgebildete Testobjekte bzw. Optotypen zu generieren, wodurch die Notwendigkeit der Anschaffung und Bereithaltung einer Vielzahl von Testscheiben, die teuer in der Herstellung und somit auch im Erwerb sind, wegfällt, sondern insbesondere eine mechanisch einfache Lösung zur Sehweitenüberprüfung bei unterschiedlichen Sehdistanzen anzugeben. Positioniert man das zweite, den Strahlengang umlenkende Element derart innerhalb des Sehtestgerätes, dass der Strahlengang zwischen dem zweiten Element und der das Testobjekt generierenden Monitoreinheit horizontal verläuft, so führt dies im Vergleich zu dem in der vorstehend zitierten Druckschrift DE 195 01 415 C2 beschriebenen Sehtestgerät zu einer erheblichen Reduzierung der vertikalen Bauhöhe des Sehtestgerätes, wodurch das Sehtestgerät nicht nur kompakter ausgebildet werden kann, sondern es wird gezielt innerhalb des Gehäuses des Sehtestgerätes Platz für eine vertikale Höhenverstellung geschaffen, durch die das gesamte Sehtestgerät auf eine individuelle Augenhöhe für den jeweiligen Probanden eingestellt werden kann. Hierdurch gewinnt das Sehtestgerät erheblich an Komfort für den zu untersuchenden Probanden, der sich in einer entspannten Körperhaltung gegenüber dem Sehtestgerät sitzend positionieren kann. Auf weitere Einzelheiten hierzu wird auf die Beschreibung des nachstehenden Ausführungsbeispieles verwiesen.

Im Wege der vorstehend beschriebenen horizontalen Orientierung des Strahlenganges zwischen dem zweiten Element und der elektronisch ansteuerbaren Monitoreinheit befindet sich die lichtemittierende Fläche der Monitoreinheit, also beispielsweise die flächige Anordnung des Pixelarrays eines LCD-Displays, senkrecht zum Strahlengang und ist demzufolge vertikal ausgerichtet. Durch diese Anordnung kann eine durch Partikelablagerung verursachte Oberflächenverschmutzung weitestgehend ausgeschlossen werden.

Aufgrund einer für Sehtestgeräte bestehenden europäischen DIN-Norm (EN ISO 8596) besteht das Erfordernis, dass die Leuchtdichte, mit der Festobjekte im Rahmen einer Augenuntersuchung in die Augen eines Probanden abgebildet werden, zwischen 80 und 320 cd/m² zu betragen hat. Um dieser Norm zu entsprechen, ist es von zentraler Bedeutung, dass die im Strahlengang des Sehtestgerätes eingesetzten optischen Komponenten möglichst keine Lichtverluste verursachen, zumal die heutige Display-Technik Digital-Displays mit den vorstehend geforderten Abmaßen und Pixelauflösungen auch im unteren Preissegment mit Leuchtdichten zwischen 80 und 100 cd/m² zu realisieren vermag. Ebenso vermögen derartige LCD-Displays nicht nur die Erzeugung monochromer Optotypen, sondern auch die Erzeugung individueller Farben, so dass die Prüfung des Sehvermögens über die reine Sehschärfebestimmung der zu untersuchenden Augen auch auf die Farbsehtüchtigkeit erweitert werden kann.

Da in aller Regel die zur Darstellung eines Testobjektes lichtemittierende Fläche der Monitoreinheit eben ausgebildet ist, sind auch die den Strahlengang umlenkenden Elemente vorzugsweise als ebene Vollspiegel oder als ebene, total reflektierende Flächen ausgebildet. Hierdurch werden jegliche optische Verzerrungen oder Doppelbilder, wie sie bei der Verwendung von Konkavspiegeln häufig auftreten, vollständig vermieden. Auch die den von der Monitoreinheit ausgehenden Strahlengang in die Augen des Probanden abbildende Abbildungseinheit, die vorzugsweise als flache Streifenlinsenanordnung ausgebildet und farbkorrigiert ist, also einen Achromat darstellt, wird das Bild des Testobjektes frei von Verzerrungen und Farbabweichungen auf die Augen des Probanden abgebildet.

Durch die verhältnismäßig kleine Baugröße der elektronisch ansteuerbaren Monitoreinheit ist es in einem besonders bevorzugten Ausführungsbeispiel möglich, peripher um die Monitoreinheit vorzugsweise vier zueinander gleich verteilt angeordnete weisses Licht emittierende LED-Lichtquellen vorzusehen, die gemeinsam mit dem Testobjekt über die Abbildungseinheit und den zwei Umlenkelementen in die Augen eines Probanden abgebildet werden. Hierdurch kann zusätzlich das Verhalten der Augen auf zusätzliches Blendlicht untersucht werden. Die Verwendung von LED's als zusätzliche Lichtquellen ist mit dem Vorteil einer nur geringen Wärmeentwicklung verbunden, wodurch auf zusätzliche Lüftererfordernisse verzichtet werden kann. Neben einer damit verminderten Geräuschentwicklung gegenüber mit Lüftern ausgestatteten Sehtestgeräten trägt der Verzicht auf jegliche Lüfter innerhalb des Sehtestgerätes erheblich zur Verringerung einer internen Geräteverschmutzung durch Partikelablagerungen an optisch relevanten Oberflächen bei.

Zusätzlich zu der an sich bekannten Verschwenkbarkeit des ersten umlenkenden Elementes innerhalb des Sehtestgerätes, das vorzugsweise als planer Vollspiegel ausgebildet ist und für eine Umlenkung der Einblickrichtung eines Probanden in einen vertikal nach unten gerichteten Strahlengangabschnitt in Richtung der Abbildungseinheit umlenkt, wodurch insbesondere für Brillen-tragende Probanden bei gleich bleibender Augenposition eine Prüfung der Fern- und Nahsichtigkeit ermöglicht wird, wobei die Blickrichtung zur Überprüfung der Fernsichtigkeit waagrecht sowie zur Überprüfung der Nahsichtigkeit gegenüber der Horizontalen schräg nach unten orientiert ist, ist zur Vergrößerung des nach unten gerichteten Blickwinkels ein Kippmechanismus vorgesehen, der das erste und zweite Element nebst Abbildungseinheit und Testobjekt gemeinsam um eine Kippachse verkippt. Die Kippachse ist hierbei horizontal orientiert und liegt in der Fokalebene, in der sich auch die Augen des Probanden befinden, so dass die Augenposition während des Kippens beibehalten werden kann. Ein Verkippen aller, für die Prüfung des Sehvermögens erforderlichen optischen sowie auch elektronischen Komponenten kann erstmals durch die Auswahl möglichst klein und kompakt bauender Einzelkomponenten, insbesondere durch die Verwendung einer elektronisch ansteuerbaren Monitoreinheit mit einem vertretbaren konstruktiven und mechanischen Aufwand realisiert werden.

Eine weitere optimale Ergänzung des Sehtestgerätes sieht die Möglichkeit einer perimetrischen Untersuchung des Gesichtsfeldes eines Probanden vor. Hierbei sind räumlich verteilt um das erste Umlenkelement in Blickrichtung des Probanden eine Vielzahl räumlich verteilter Lichtquellen angeordnet, beispielsweise in Form von weisses Licht emittierenden LED's. Zur Fixierung der Blickrichtung wird ausgehend von der Monitoreinheit eine Zentriermarke auf das erste Umlenkelement mittig zur Blickrichtung abgebildet. Auf weitere Einzelheiten wird auf die nachfolgende Beschreibung unter Bezugnahme auf das Ausführungsbeispiel verwiesen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: schematisierter Querschnitt durch die optischen Komponenten eines Sehtestgerätes;

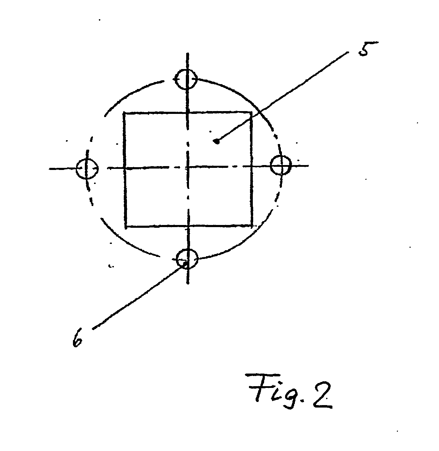

- Fig. 2: schematisierte Ansicht einer elektronisch ansteuerbaren Monitoreinheit mit peripher um diese angeordneten Lichtquellen,
- Fig. 3: schematisierte Darstellung des Kippmechanismus sowie
- Fig. 4: schematisierte Darstellung zur Perimeteranordnung.

### Wege zur Ausführung der Erfindung, gewerbliche Verwendbarkeit

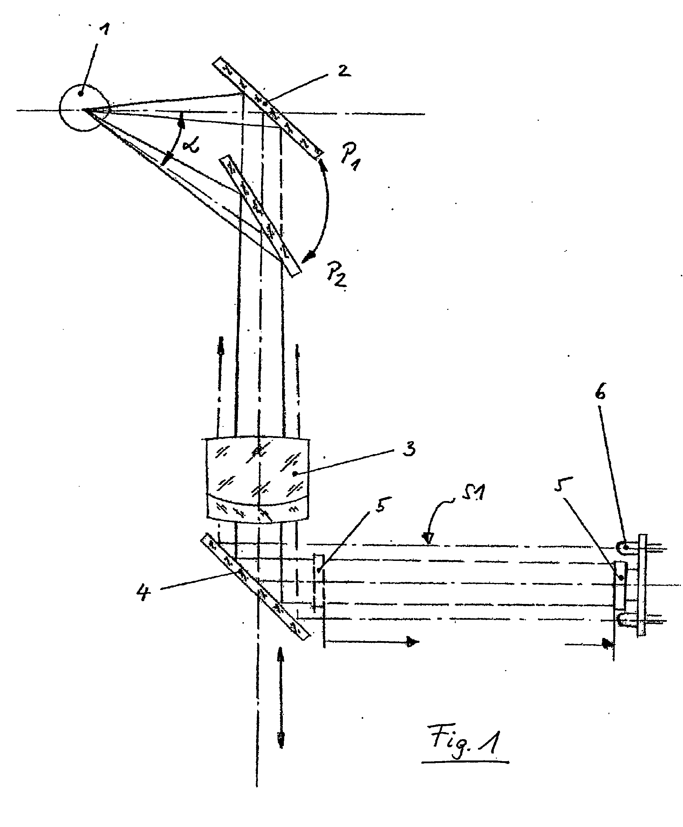

Fig. 1 zeigt schematisiert alle optisch erforderlichen Komponenten zur Prüfung des Sehvermögens der Augen eines Probanden. So setzt sich das Sehtestgerät im wesentlichen durch die in Blickrichtung der zu untersuchenden Augen 1 nachgeordneten folgenden Komponenten zusammen: Ein als ebener Vollspiegel ausgebildetes erstes Umlenkelement 2, eine farbkorrigierte Abbildungseinheit 3, ein stationär gegenüber der Abbildungseinheit 3 angeordnetes zweites Umlenkelement 4, das gleichsam dem ersten Umlenkelement 2 als ebener Vollspiegel ausgebildet ist und letztlich eine elektronisch ansteuerbare Monitoreinheit 5, die längs des durch das zweite Umlenkelement 2 umgelenkten Strahlengang S1 linear verschieblich angeordnet ist.

Das zweite Umlenkelement 4 lenkt den von der Monitoreinheit 5 ausgehenden Strahlengang S1, der waagrecht bzw. horizontal orientiert ist, um 90° vertikal nach oben durch die Abbildungseinheit 3 ab. Durch die längs des Strahlenganges S1 linear verschiebbar angeordnete, kompakt ausgebildete Monitoreinheit 5 ist es möglich, eine optische Sehweitenvariation zwischen den zu untersuchenden Augen und der Monitoreinheit von typischerweise 33 cm bis unendlich und darüber hinaus für eine Hyperopieprüfung durchzuführen, ohne die Notwendigkeit jeglicher weiterer optischer Komponenten. Insbesondere bleibt der vertikal unterhalb des zweiten Umlenkelementes 2 befindliche Raumbereich, im Gegensatz zu dem in der DE 195 01 415 C2 beschriebenen Sehtestgerätes, vollständig unverbaut, so dass hierdurch die Möglichkeit geschaffen ist, innerhalb eines nicht weiter dargestellten, die optischen und elektronischen Komponenten umfassenden Gehäuses, eine vertikale Höhenverstellung vorzusehen, durch die die in Fig. 1 dargestellten optischen und elektronischen Komponenten vertikal individuell an die Augenhöhe des Probanden 1 angepasst werden können. So wird das Sehtestgerät in den meisten Fällen auf einem Tisch platziert, gegenüber dem sich ein Proband auf einen nicht notwendigerweise höhenverstellbaren Stuhl setzt. Durch eine im Sehtestgerät selbst vorgesehene vertikale Höhenverstellung, die vorzugsweise unterhalb der in Fig.1 dargestellten Komponenten innerhalb des Gehäuses des Sehtestgerätes angeordnet ist, können Probanden mit unterschiedlichen Augenhöhen bei jeweils bequemer Sitzhaltung untersucht werden.

Die elektronische ansteuerbare Monitoreinheit 5 ist vorzugsweise als Farb-LCD-Display ausgebildet und ermöglicht die Generierung von Optotypen in beliebiger Form, Größe und Farbe. Als Display-Größen eignen sich 10 x 10 mm große Display-Flächen, die jeweils quadratische Pixelgrößen von etwa 15 µm Kantenlänge aufweisen mit einer Auflösung von wenigstens 800 x 600 Pixel. Mit derartigen Displays sind typische Optotypen mit der für die Sehtestprüfung erforderlichen Konturenschärfe generierbar, wie beispielsweise Landold-Ringe, Zahlen oder Kindersehzeichen sowie auch Stereoteste mit Rot/Grün-Trennung, Kontrastzeichen mit oder ohne Blendlichtwirkung sowie auch Farbteste.

Zur Untersuchung der Blendlichtwirkung auf die Augen 1 eines Probanden sind um die Monitoreinheit 5 weisses Licht emittierende LED's 6 angeordnet, gemäß der in Fig. 1 dargestellten Anordnung. Die Verwendung von LED's ebenso wie der Einsatz eines LCD-Displays hat den Vorteil einer einfachen elektronischen Verschaltung sowie einer Niederspannungsversorgung, sodass die Stromversorgung der elektronischen Komponenten sowie die damit verbundene Wärmeentwicklung eine nur untergeordnete Rolle spielt.

Wie bereits erwähnt, können auf Lüfteranordnungen und die damit verbundenen Nachteile vollständig verzichtet werden.

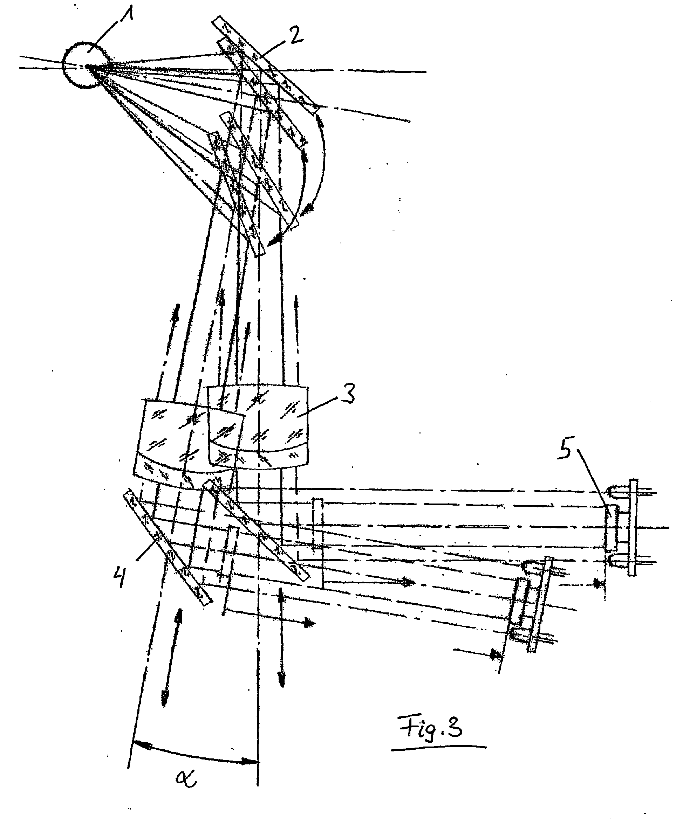

In Fig. 1 sind zwei Stellpositionen des ersten Umlenkelementes 2 dargestellt, eine erste Position P1, in der die Blickrichtung des Probanden horizontal bzw. waagrecht orientiert ist und das Bild des Testobjektes, das durch die Abbildungseinheit 3 vertikal von unten auf die Umlenkeinheit 2 auftrifft, um 90 ° in die Augen des Probanden 1 umgelenkt wird. In einer zweiten Position P2 blickt der Proband um einen Winkel α gegenüber der Horizontalen nach unten. Auch in dieser Position gelangt das Bild des Testobjektes über die Abbildungseinheit 3 und der sich in Position P2 befindlichen Umlenkeinheit 2 in das Auge 1 des Probanden. Die Verschwenkung des Umlenkelementes 2 in die Position P2 ist für die Untersuchung von Brillen-tragenden Probanden erforderlich, die über unterschiedliche Sehkorrekturen für den Fern- und Nahbereich verfügen. Um zu vermeiden, dass die Fläche des Umlenkelementes 2 unverhältnismäßig groß ausgebildet ist, begrenzt sich der Winkel zur Blickwinkelabsenkung, d.h. die maximal mögliche Verschwenkung der Umlenkeinheit 2 typischerweise auf α = 35°. Modernere Gleitsichtbrillen ermöglichen einem Träger jedoch Blickwinkelabsenkungen, ohne den Kopf zu bewegen, von bis zu 45°. Um auch jene abgesenkten Blickwinkelbereiche mit Hilfe des Sehtestgerätes überprüfen zu können, sieht eine optionale Ausbildung des Sehtestgerätes einen Kippmechanismus vor, der in Fig. 3 dargestellt ist. So ermöglicht eine Verkippung der gesamten Komponenten zur Untersuchung des Sehvermögens eines Probanden um eine Kippachse, die horizontal durch die Fokalebene der Abbildungseinheit 3 verläuft, eine weitere Vergrößerung des Kippwinkels α, so dass auch größte Blickwinkelabsenkungen mit Hilfe des Sehtestgerätes erfassbar sind.

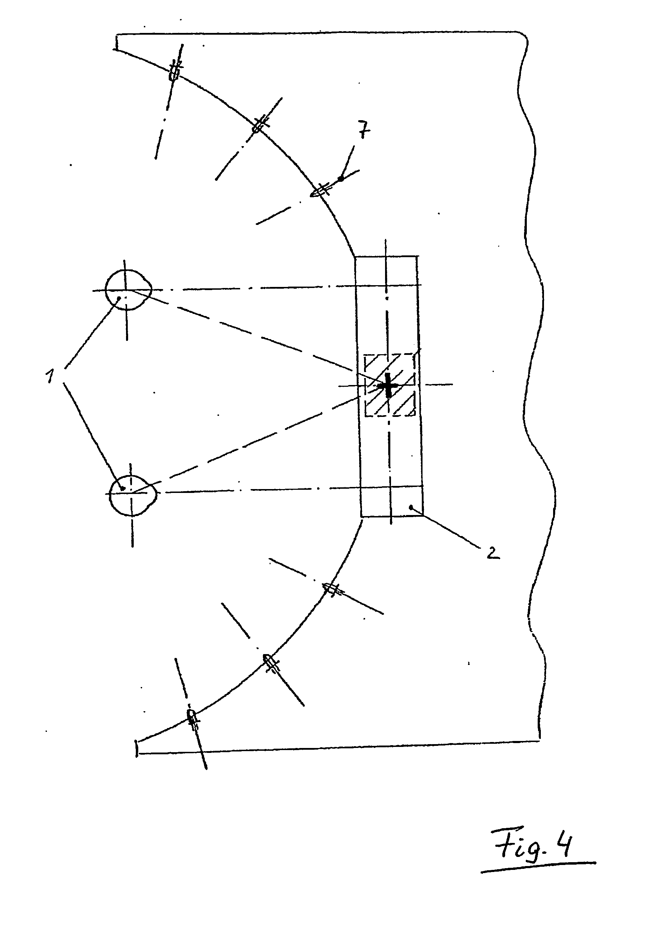

Letztlich sieht Fig. 4 eine weitere optionale Erweiterung des Sehtestgerätes vor, mit der die Augen 1 perimetrisch untersucht werden können. Hierzu ist in Blickrichtung der Augen 1 um das erste Umlenkelement 2 räumlich verteilt eine Vielzahl von Lichtquellen in Form von LED's 7 angeordnet. Zur Festlegung und Fixierung der Blickrichtung ist mittig zur Blickrichtung das Testobjekt 5 mit einer vom Untersucher individuell positionierbaren Zentriermarke vorgesehen, die in einer bevorzugten Ausführungsform mit Hilfe des LCD-Displays 5 generierbar ist.

Reihenhaft sei nachfolgend die mit dem erfindungsgemäß ausgebildeten Sehtestgerät verbundenen Vorteile genannt:

Mit Hilfe der modernen Farb-Display-Technik ist die unabhängige Sehzeichengenerierung mit Hilfe geeigneter Display-Ansteuerungssoftware möglich. Auf diese Weise können sowohl zeitlich stationäre Optotypen generiert werden sowie auch dynamische, sich zeitlich verändernde Testobjekte dargestellt werden, um letztlich eine dynamische Sehschärfenbestimmung durchführen zu können. Durch die Substitution der bislang eingesetzten hochpreisigen Durchlichttestscheiben, die mittels Schrittmotortechnik angesteuert werden, ist durch die neuartige Display-Technologie ein entscheidender Faktor zur Kosten- und Gewichtsreduzierung verbunden. Die Display-Technologie sowie die Verwendung von LED-Lichtquellen zur Erzeugung von Blendlicht erfordern Niederspannungsnetzteile, die nur über geringes Gewicht verfügen und keinerlei Lüftertechnik benötigen. Hierdurch ist eine erhebliche Leistungseinsparung möglich, die sich letztlich in Betriebs- sowie auch Wartungskosten positiv niederschlagen. Durch die kompakte Bauform des Sehtestgerätes wird Platz innerhalb des Gerätes gewonnen, der für eine individuelle Höhenverstellung an die jeweilige Sitzhöhe des Probanden nutzbar ist.

### Bezugszeichenliste

- 1: Augen
- 2: Umlenkelement
- 3: Abbildungseinheit
- 4: Umlenkelement
- 5: Monitoreinheit
- 6: Blendlichtquellen
- 7: LED's

## Patentansprüche

1. Sehtestgerät zur Prüfung des Sehvermögens der Augen (1) eines Probanden mit einer optischen Abbildungseinheit (3) zur virtuellen Abbildung eines innerhalb der Brennweite der Abbildungseinheit (3) befindlichen und aus unterschiedlichen Entfernungen abbildbaren Testobjekts wobei ein von den in der Fokalebene der Abbildungseinheit (3) befindlichen Augen (1) des Probanden bedachtbares virtuelles Bild des Testobjekts erzeugt wird, und mit einem im Strahlengang zwischen den Augen (1) des Probanden und der Abbildungseinheit (3) angeordneten ersten optischen Element (2) sowie einem zwischen der Abbildungseinheit (3) und dem Testobjekt angeordneten zweiten optischen Element (4),
**dadurch gekennzeichnet, dass** das erste und das zweite optische Element (2, 4) als Vollspiegel oder als ein den Strahlengang totalreflektierendes optisches Element ausgebildet sind, wobei das zweite optische Element (4) relativ zur Abbildungseinheit (3) im Strahlengang stationär angeordnet ist, und
dass das Testobjekt durch eine elektronisch ansteuerbare Monitoreinheit (5) erzeugbar ist, die längs des durch das zweite optische Element (4) umgelenkten Strahlengangs (S1) beweglich angeordnet ist.

2. Sehtestgerät nach Anspruch 1,
**dadurch gekennzeichnet, dass** das erste und das zweite optische Element (2, 4) ein planer oder gekrümmter Vollspiegel ist oder eine Prismenfläche aufweist, die unter dem Brewster-Winkel relativ zum Strahlengang angeordnet ist.

3. Sehtestgerät nach Anspruch 1 oder 2,
**dadurch gekennzeichnet, dass** das zweite optische Element (2, 4) den Strahlengang derart umlenkt, dass der Strahlengang (S1) zwischen dem zweiten optischen Element (4) und dem Testobjekt horizontal orientiert ist.

4. Sehtestgerät nach Anspruch 3,
**dadurch gekennzeichnet, dass** das zweite optische Element (4) den Strahlengang um 90° umlenkt.

5. Sehtestgerät nach einem der Ansprüche 1 bis 4,
**dadurch gekennzeichnet, dass** das erste optische Element (2) in wenigstens zwei Stellungen (P1, P2) überführbar ist, eine erste Stellung, (P1) in der bei horizontaler Blickrichtung des Probanden das erste optische Element (2) den Strahlengang um 90° umlenkt, und eine zweite Stellung (P2), in der bei gegenüber der Horizontalen nach unten geneigten Blickrichtung des Probanden das erste optische Element (2) den Strahlengang um größer 90° umlenkt,
so dass in beiden Stellungen (P1, P2) des ersten optischen Elementes (2) der Strahlengang zwischen dem ersten optischen Element (2) und der Abbildungseinheit (3) vertikal orientiert ist.

6. Sehtestgerät nach einem der Ansprüche 1 bis 5,
**dadurch gekennzeichnet, dass** die Monitoreinheit (5) eine zur Darstellung eines Testobjektes lichtemittierende Fläche aufweist, deren Abbild über das zweite optische Element (4), der Abbildungseinheit (3) sowie über das erste optische Element (2) in die Fokalebene abbildbar ist.

7. Sehtestgerät nach Anspruch 6,
**dadurch gekennzeichnet, dass** die lichtemittierende Fläche vertikal angeordnet ist.

8. Sehtestgerät nach einem der Ansprüche 1 bis 7,
**dadurch gekennzeichnet, dass** die Monitoreinheit (5) als lichtemittierende Fläche ein LCD-Display aufweist.

9. Sehtestgerät nach Anspruch 8,
**dadurch gekennzeichnet, dass** das LCD-Display eine Größe von etwa 10 x 10 mm mit einer Pixeldichte von wenigstens 600 x 800 aufweist.

10. Sehtestgerät nach einem der Ansprüche 1 bis 8,
**dadurch gekennzeichnet, dass** die Monitoreinheit (5) das Testobjekt mit einer Leuchtdichte von wenigstens 80 cd/m² emittiert.

11. Sehtestgerät nach einem der Ansprüche 1 bis 10,
**dadurch gekennzeichnet, dass** die Abbildungseinheit (3) in Art einer Streifenlinsenanordnung ausgebildet und farbkorrigiert ist.

12. Sehtestgerät nach einem der Ansprüche 1 bis 11,
**dadurch gekennzeichnet, dass** eine vertikale Höhenverstellung vorgesehen ist, durch die zur Höhenanpassung an die Augenhöhe des Probanden, das erste und zweite optische Element (2, 4) nebst Abbildungseinheit (3) und Testobjekt gemeinsam vertikal höhenverstellbar sind.

13. Sehtestgerät nach einem der Ansprüche 6 bis 12,
**dadurch gekennzeichnet, dass** in der Ebene der lichtemittierenden Fläche peripher um die Monitoreinheit (5) wenigstens eine zusätzliche Lichtquelle (6), die als Blendlichtquelle dient, angeordnet ist.

14. Sehtestgerät nach Anspruch 13,
**dadurch gekennzeichnet, dass** wenigstens zwei Lichtquellen (6) um die lichtemittierende Fläche äquidistant zueinander angeordnet sind.

15. Sehtestgerät nach Anspruch 13 oder 14,
**dadurch gekennzeichnet, dass** die Lichtquelle (6) eine weisses Licht emittierende LED ist.

16. Sehtestgerät nach einem der Ansprüche 1 bis 15,
**dadurch gekennzeichnet, dass** ein Kippmechanismus vorgesehen ist, der das erste und zweite optische Element (2, 4) nebst Abbildungseinheit (3) und Testobjekt gemeinsam um eine Kippachse verkippt.

17. Sehtestgerät nach Anspruch 16,
**dadurch gekennzeichnet, dass** die Kippachse horizontal orientiert ist und in der Fokalebene liegt.

18. Sehtestgerät nach einem der Ansprüche 1 bis 17,
**dadurch gekennzeichnet, dass** das Testobjekt eine Zentriermarke aufweist, die mittig zu den Augen (1) des Probanden abbildbar ist, und
dass peripher um das erste optische Element (2) eine Vielzahl von Lichtquellen angeordnet ist.

19. Sehtestgerät nach Anspruch 18,
**dadurch gekennzeichnet, dass** die Vielzahl der Lichtquellen in einer, einen Halbraum um die Augen in Blickrichtung umschließenden gekrümmtem Fläche angeordnet sind.

20. Sehtestgerät nach Anspruch 18 oder 19,
**dadurch gekennzeichnet, dass** die Zentriermarke mittels der elektronisch ansteuerbare Monitoreinheit (5) erzeugbar ist.

## Claims

1. A vision-testing device for checking the ocular perception of the eyes (1) of a test person, the device having an optical imaging unit (3) for virtual imaging of a test object which is located within the focal length of the imaging unit (3) and which is imageable from various distances, with a virtual image of the test object being generated which is observable by one of the test person's eyes which is located in the focal plane of the imaging unit (3), and having a first optical element (2) placed in the beam path between the eyes (1) of the test person and the imaging unit (3) and a second optical element (4) placed between the imaging unit (3) and the test object,
wherein the first and second optical element (2,4) are constructed as standard mirrors or as an optical element which reflects the beam path totally, with the second optical element (4) being placed stationary relative to the imaging unit (3) in the beam path, and
the test object is producible by an electronically triggerable monitoring unit (5) which is moveably disposed along the beam path (S1) deflected by the second optical element (4).

2. The vision-testing device according to claim 1,
wherein the first and the second optical element (2,4) are a plane or a curved standard mirror or have a prism surface which is disposed at a Brewster's angle relative to the beam path.

3. The vision-testing device according to claim 1 or 2,
wherein the second optical element (2,4) deflects the beam path (4) in such a manner that the beam path (S1) is oriented horizontally between the second optical element (4) and the test object.

4. A vision-testing device according to claim 3,
**wherein** the second optical element (4) deflects the beam path 90°.

5. The vision-testing device according to one of the claims 1 to 4,
wherein the first optical element (2) can be conveyed into at least two positions (P1 ,P2), a first position (P1), in which if the test person's line of vision is horizontal the first optical element (2) deflects the beam path 90°, and a second position (P2), in which if the test person's line of vision is inclined downward in relation to the horizontal line, the first optical element (2) deflects the beam path more than 90°,
in such a manner that in both positions (P1,P2) of the first optical element (2) the beam path is oriented vertically between the first optical element (2) and the imaging unit (3).

6. The vision-testing device according to one of the claims 1 to 5,
wherein the monitoring unit (5) has a light-emitting surface for representation of a test object whose image is imageable in the focal plane via the second optical element (4), the imaging unit (3) and the first optical element (2).

7. The vision-testing device according to claim 6,
wherein the light-emitting surface is placed vertically.

8. The vision testing device according to one of the claims 1 to 7,
wherein the monitoring unit (5) has a LCD display as the light-emitting surface.

9. The vision-testing device according to claim 8,
**wherein** the dimensions of the LCD display are approximately 10 x 10 mm with a pixel density of at least 600 x 800.

10. The vision-testing device according to one of the claims 1 to 8,
wherein the monitoring device (5) emits the test object with an illumination density of at least 80 cd/m².

11. The vision-testing device according to one of the claims 1 to 10,
wherein the imaging unit (3) is designed in the manner of a strip lens arrangement and is color corrected.

12. The vision-testing device according to one of the claims 1 to 11,
wherein vertical height adjustment is provided by means of which the first and the second optical element (2,4) as well as the imaging unit (3) and the test object are vertically adjustable together in height for height adjustment to the test person's eye level.

13. The vision-testing device according to one of the claims 6 to 12,
**wherein** in the plane of the light-emitting surface, at least one additional light source (6) which acts as a glare source is disposed peripherally about the monitoring unit (5).

14. The vision-testing device according to claim 13,
wherein at least two light sources (6) are placed about the light-emitting surface equidistant to each other.

15. The vision-testing device according to claim 13 or 14,
wherein the light source (6) is a white-light-emitting LED.

16. The vision-testing device according to one of the claims 1 to 15,
wherein a tilting mechanism is provided which tilts the first and the second optical element (2,4) as well as the imaging unit (3) and the test object together about a tilting axis.

17. The vision-testing device according to claim 16,
wherein the tilting axis is oriented horizontally and lies in the focal plane.

18. The vision-testing device according to one of the claims 1 to 17,
wherein the test object is provided with a centering mark which is imageable centered to the test person's eyes (1) and
a multiplicity of light sources is disposed peripherally about the first optical element (2).

19. The vision-testing device according to claim 18,
wherein the multiplicity of light sources is disposed in a curved surface enclosing a half-space about the eyes in the line of vision.

20. The vision-testing device according to claim 18 or 19,
wherein the centering mark is generatable by means of the electronically triggerable monitoring unit (5).

## Revendications

1. Optomètre pour contrôler l'acuité visuelle des yeux (1) d'un sujet avec une unité de reproduction optique (3) pour reproduire virtuellement un élément de test se trouvant à l'intérieur de la focale de l'unité de reproduction optique (3) et pouvant être reproduit depuis différentes distances, moyennant quoi une image virtuelle concevable de l'élément de test est produite depuis les yeux (1) du sujet se trouvant dans le plan focal de l'unité de reproduction (3) et avec un premier élément optique (2) placé dans la trajectoire du faisceau entre les yeux (1) du sujet et l'unité de reproduction optique (3) ainsi qu'un deuxième élément optique (4) placé entre l'unité de reproduction (3) et l'élément de test, **caractérisé en ce que** les premier et deuxième éléments optiques (2,4) sont formés comme un miroir plein ou comme un élément optique réfléchissant totalement la trajectoire du faisceau, le deuxième élément optique (4) étant placé de manière fixe dans la trajectoire du faisceau par rapport à l'unité de reproduction optique (3), et **en ce que** l'élément de test peut être produit par une unité de surveillance (5) commandable électroniquement, qui est placée de manière mobile le long de la trajectoire du faisceau (S1) déviée par le deuxième élément optique (4).

2. Optomètre selon la revendication 1, **caractérisé en ce que** les premier et deuxième éléments optiques (2,4) sont un miroir plein plat ou courbe ou présentent une surface de prismes qui est placée sous l'angle de Brewster par rapport à la trajectoire du faisceau.

3. Optomètre selon la revendication 1 ou 2, **caractérisé en ce que** le deuxième élément optique (4) dévie la trajectoire du faisceau de manière à ce que la trajectoire du faisceau (S1) soit orientée horizontalement entre le deuxième élément optique (4) et l'élément de test.

4. Optomètre selon la revendication 3, **caractérisé en ce que** le deuxième élément optique (4) dévie la trajectoire du faisceau de 90°.

5. Optomètre selon l'une des revendications 1 à 4, **caractérisé en ce que** le premier élément optique (2) peut être amené dans au moins deux positions (P1, P2), une première position (P1) dans laquelle avec une direction de visée horizontale du sujet le premier élément optique (2) dévie la trajectoire du faisceau de 90°, et une deuxième position (P2), dans laquelle avec une direction de visée du sujet dirigée vers le bas par rapport à l'horizontale, le premier élément optique (2) dévie la trajectoire du faisceau de plus de 90°, de façon à ce que dans les deux positions (P1, P2) du premier élément optique (2), la trajectoire du faisceau soit orientée verticalement entre le premier élément optique (2) et l'unité de reproduction optique (3).

6. Optomètre selon l'une des revendications 1 à 5, **caractérisé en ce que** l'unité de surveillance (5) présente une surface émettant de la lumière pour représenter l'élément de test, dont la reproduction peut être reproduite dans le plan focal par le deuxième élément optique (4) de l'unité de reproduction optique (3) ainsi que par le premier élément optique (2).

7. Optomètre selon la revendication 6, **caractérisé en ce que** la surface émettant de la lumière est placée à la verticale.

8. Optomètre selon l'une des revendications 1 à 7, **caractérisé en ce que** l'unité de surveillance (5) présente un écran à cristaux liquides comme surface émettant de la lumière.

9. Optomètre selon la revendication 8, **caractérisé en ce que** l'écran à cristaux liquides présente une taille d'environ 10 x 10 mm avec une résolution de pixels de 600 x 800.

10. Optomètre selon l'une des revendications 1 à 8, **caractérisé en ce que** l'unité de surveillance (5) émet l'élément de test avec une luminance d'au moins 80 cd/m².

11. Optomètre selon l'une des revendications 1 à 10, **caractérisé en ce que** l'unité de reproduction (3) est formée ou corrigée chromatiquement dans une sorte de disposition de lentilles à bandes.

12. Optomètre selon l'une des revendications 1 à 11, **caractérisé en ce qu'**un réglage vertical de la hauteur est prévu par lequel pour adapter la hauteur à la hauteur des yeux du sujet, les premier et deuxième éléments optiques (2,4) sont, outre l'unité de reproduction optique (3) et l'élément de test, conjointement réglables en hauteur verticalement.

13. Optomètre selon l'une des revendications 6 à 12, **caractérisé en ce** dans le plan de la surface émettant de la lumière, au moins une source lumineuse (6) supplémentaire qui sert de source lumineuse d'éblouissement, est placée sur le périmètre de l'unité de surveillance (5).

14. Optomètre selon la revendication 13, **caractérisé en ce qu'**au moins deux sources lumineuses (6) sont placées à équidistance l'une de l'autre autour de la surface émettant de la lumière.

15. Optomètre selon l'une des revendications 13 ou 14, **caractérisé en ce que** la source lumineuse est une DEL émettant une lumière blanche.

16. Optomètre selon l'une des revendications 1 à 15, **caractérisé en ce qu'**un mécanisme de bascule est prévu qui bascule les premier et deuxième éléments optiques (2,4) conjointement sur un axe de basculement avec l'unité de reproduction optique (3) et l'élément de test.

17. Optomètre selon la revendication 16, **caractérisé en ce que** l'axe de basculement est orienté horizontalement et figure dans le plan focal.

18. Optomètre selon l'une des revendications 1 à 17, **caractérisé en ce que** l'élément de test présente un marquage central, qui peut être reproduit de manière centrale par rapport aux yeux (1) du sujet et qu'une pluralité de sources lumineuses est placée sur le périmètre du premier élément optique (2).

19. Optomètre selon la revendication 18, **caractérisé en ce que** la pluralité de sources lumineuses est placée sur une surface courbe délimitant un espace semi-fini autour des yeux dans la direction de visée.

20. Optomètre selon l'une des revendications 18 ou 19, **caractérisé en ce que** le marquage central peut être produit au moyen de l'unité de surveillance (5) commandable électroniquement.
